# DEMANDE DE BREVET EUROPEEN

(11) **EP 0 598 652 A1**
(43) Date de publication de la demande: **25.05.1994**
(21) Numéro de dépôt: 93402765.7
(22) Date de dépôt: 15.11.1993
(51) Int. Cl.: C02F 1/02, A61L 2/04

(54) **Appareil perfectionné pour la production d'eau stérile chaude ou froide à partir d'eau de ville**

(30) Priorité: 16.11.1992 FR 9213739
(71) Demandeur: EQUIP'TECHNIC, F-77440 Lizy-sur-Ourcq (FR)
(72) Inventeur: Baeckeroot, Georges, F-77910 Germigny l'Eveque (FR)
(74) Mandataire: Schrimpf, Robert

(57) **Abrégé**

L'invention concerne un appareil pour la production d'eau stérile (ES) à partir d'eau de ville (EV), du type comprenant un circuit d'eau comportant une canalisation amont (1) alimentée en eau de ville, traversant un échangeur de chaleur (2) et débouchant dans un réservoir (3) muni d'une résistance électrique (5) apte à porter l'eau du réservoir à une température de stérilisation et une canalisation aval (6) partant du réservoir, traversant l'échangeur (2) et aboutissant à une sortie d'eau stérile (ES) munie d'un robinet de distribution (7). L'échangeur de chaleur (2) est apte à réaliser un échange de chaleur entre ladite canalisation aval (1) et ladite canalisation amont (6). L'appareil comporte un circuit de circulation d'eau chaude stérile comportant une canalisation de circulation (8) partant de la canalisation aval à un point de prélèvement (9) situé entre l'échangeur de chaleur (2) et le robinet de distribution (7) et débouchant dans le réservoir (3) et une pompe (10) montée dans cette canalisation pour y faire circuler l'eau chaude stérile prélevée dans la canalisation aval (6) lorsque le robinet de distribution (7) est fermé.

## Description

La présente invention concerne un appareil pour la production d'eau stérile à partir d'eau de ville, afin d'alimenter en eau stérile froide ou chaude et en continu des installations de distribution, notamment dans les hôpitaux, par exemple des lavabos, douches ou bains destinés aux grands brûlés, aux nouveaux-nés, aux malades immunodépressifs... .

On connaît, par la publication FR-2 627 479, un appareil capable de fournir en continu de l'eau stérile froide. Cet appareil comprend un premier circuit d'eau comportant en série une canalisation amont comportant une entrée d'eau de ville, une électovanne, et débouchant dans un réservoir muni d'une résistance électrique apte à porter l'eau du réservoir à une température de stérilisation et une canalisation aval d'échange de chaleur partant du réservoir et aboutissant à une sortie d'eau stérile, et un deuxième circuit d'eau comportant en série une canalisation d'alimentation munie d'une entrée d'eau de ville et d'une électrovanne, une canalisation d'échange de chaleur en situation d'échange de chaleur avec ladite canalisation aval du premier circuit et débouchant dans ledit réservoir.

Un tel appareil ne donne cependant pas entièrement satisfaction. Ainsi, en dehors des périodes de distribution d'eau stérile, la canalisation aval aboutissant à la sortie d'eau stérile se refroidit et l'eau qu'elle contient n'est plus maintenue dans des conditions stériles. Par ailleurs, cet appareil connu ne permet pas de régler la température de l'eau stérile qu'il délivre, ni son débit.

L'invention a pour objet de réaliser un appareil perfectionné remédiant aux inconvénients précités.

L'appareil selon l'invention, pour la production d'eau stérile à partir d'eau de ville, est du type comprenant un circuit d'eau comportant une canalisation amont alimentée en eau de ville, traversant un échangeur de chaleur et débouchant dans un réservoir muni d'une résistance électrique apte à porter l'eau du réservoir à une température de stérilisation et une canalisation aval partant du réservoir, traversant l'échangeur et aboutissant à une sortie d'eau stérile munie d'un robinet de distribution, ledit échangeur de chaleur étant apte à réaliser un échange de chaleur entre la canalisation aval et la canalisation amont.

Selon une caractéristique de l'invention, l'appareil comporte un circuit de circulation d'eau chaude stérile comprenant une canalisation de circulation partant de ladite canalisation aval à un point de prélèvement situé entre l'échangeur de chaleur et le robinet de distribution, et débouchant dans le réservoir et une pompe montée dans cette canalisation, pour y faire circuler l'eau chaude stérile prélevée dans la canalisation aval lorsque le robinet de distribution est fermé.

L'invention permet ainsi de stériliser la canalisation aval et le robinet de distribution lors d'une première utilisation de l'appareil et de garder l'eau contenue dans la canalisation aval dans des conditions stériles lorsque le robinet de distribution est fermé.

Avantageusement, la canalisation amont est alimentée en eau de ville au moyen d'un mitigeur comportant une entrée d'eau chaude sanitaire et une entrée d'eau de ville, de façon à permettre un réglage de la température de l'eau de ville alimentant la canalisation amont et la température de sortie de l'eau stérile.

Avantageusement, l'appareil comporte dans la canalisation amont une vanne de réglage du débit d'alimentation en eau de ville de cette canalisation.

Avantageusement, l'appareil comporte, partant de la canalisation de circulation, en aval de la pompe et en amont d'un clapet anti-retour monté dans la canalisation de circulation, un circuit de purge muni d'une électrovanne débouchant sur une canalisation d'évacuation vers un égout, ainsi qu'une alimentation en eau froide pour refroidir l'eau délivrée par cette électrovanne lors de son rejet dans ladite canalisation d'évacuation.

Avantageusement, l'appareil comporte des premiers moyens de commande pour déclencher l'évacuation de l'eau chaude stérile contenue dans la canalisation de circulation et dans la canalisation aval vers l'égout, préalablement à l'ouverture du robinet de distribution.

Avantageusement, l'appareil comporte des seconds moyens de commande aptes à interdire l'ouverture du robinet de distribution lorsque l'eau contenue dans la canalisation aval à la sortie de l'échangeur est à une température supérieure à une valeur donnée.

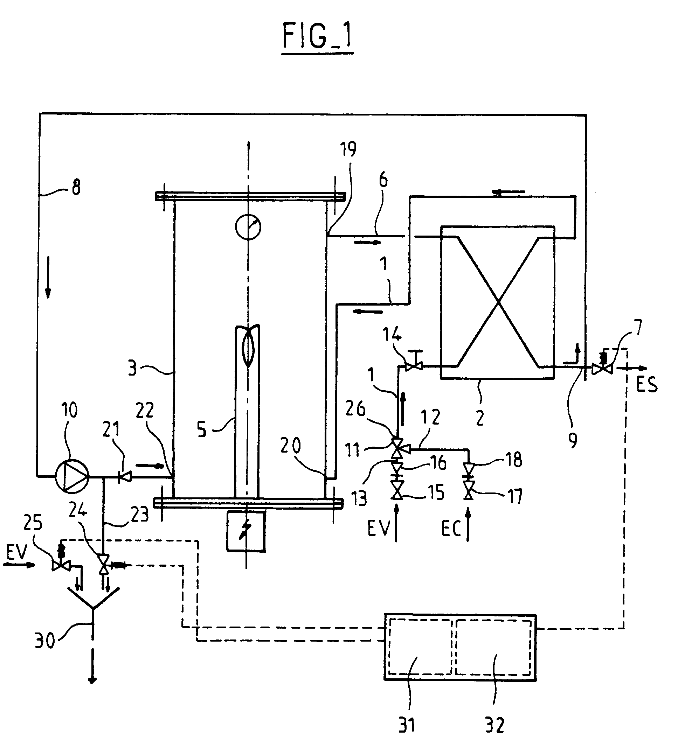

On décrira ci-après un exemple de réalisation de l'appareil selon l'invention, en référence à la figure unique du dessin joint qui en montre le schéma, et son procédé d'utilisation.

L'appareil est constitué par un ensemble monobloc qui comprend:
. un circuit de production d'eau chaude stérile comprenant une canalisation amont 1 reliée à la sortie 26 d'un mitigeur11 comportant une entrée 13 alimentée au travers d'une vanne 15 et d'un clapet anti-retour 16 (sens passant vers le mitigeur 11) par une source d'eau de ville EV à la température d'environ 15°C et une entrée 12 alimentée au travers d'une vanne 17 et d'un clapet anti-retour 18 (sens passant vers le mitigeur 11) par une source d'eau chaude EC sanitaire à la température d'environ 60°C. La canalisation amont 1 traverse un échangeur de chaleur 2 et débouche par une entrée basse 20 dans un réservoir 3. Une vanne 14 de réglage du débit est placée dans la canalisation amont 1 en aval du mitigeur 11 et en amont de l'échangeur 2. L'échangeur de chaleur 2 est prévu pour réchauffer l'eau de ville circulant dans la canalisation amont 1 avant qu'elle n'alimente l'entrée 20 du réservoir 3, comme cela sera vu dans la suite. Une canalisation aval 6 part d'une sortie haute 19 prévue sur le réservoir 3, passe dans l'échangeur de chaleur 2 et débouche sur une sortie d'eau stérile ES munie d'un robinet de distribution 7, de préférence constitué par une électrovanne.

Le réservoir 3 est par exemple une cuve de 10 litres environ, équipée d'une résistance électrique 5 de 5 kW qui permet de chauffer l'eau à une température de 90°C environ pour la stériliser. La sortie haute 19 et l'entrée basse 20 sont éloignées l'une de l'autre de façon à ce que le temps de séjour de l'eau dans le réservoir soit suffisant pour assurer sa stérilisation.

L'échangeur de chaleur 2 est une enceinte dans laquelle la canalisation aval 6 et la canalisation amont 1 sont en situation d'échange de chaleur. L'échangeur 3 peut être constitué de plaques ou de tubes suivant la quantité de chaleur à échanger. L'échangeur 2 est conçu, lors de l'utilisation de l'appareil pour :
. refroidir l'eau stérile produite par le réservoir 3 jusqu'à une température dépendant du réglage du mitigeur 11,
. préchauffer l'eau de ville d'alimentation du réservoir 3 de stérilisation, de préférence jusqu'à 80°C environ.

Conformément à l'invention, l'appareil comporte un circuit de circulation comprenant une canalisation de circulation 8 pour prélever de l'eau stérile à un point de prélèvement 9 situé sur la canalisation aval 6 en aval de l'échangeur de chaleur 2, de préférence à proximité du robinet 7, et une pompe 10 montée dans cette canalisation de circulation pour la renvoyer dans le réservoir 3. Un clapet anti-retour 21 (sens passant vers le réservoir) est placé en aval de la pompe 10 et débouche dans le réservoir 3 par une entrée basse 22, située près du fond du réservoir. Grâce au circuit de circulation, lors des périodes de non utilisation de l'appareil, c'est-à-dire lorsque le débit d'eau stérile qui s'écoule par le robinet 7 est nul, on maintient la canalisation aval 6 à une température proche de la température de l'eau dans le réservoir, 85°C environ, de sorte que l'eau contenue dans la canalisation aval reste stérile.

L'évacuation de l'eau chaude stérile contenue dans le circuit de circulation et dans la canalisation aval par une canalisation d'évacuation 30 vers un égout s'effectue à l'aide d'un circuit de purge 23 partant d'un point de la canalisation de circulation 8 situé entre la pompe 10 et le clapet anti-retour 21, et muni en sortie d'une électrovanne 24. De préférence, comme représenté, un appoint d'eau froide est réalisé au moyen d'une électrovanne 25 alimentée en eau de ville, pour abaisser lors de son rejet la température de l'eau chaude délivrée par l'électrovanne 24, de préférence à une température inférieure à 30°C environ.

De préférence, il est prévu un système de protection qui évite toute fausse manoeuvre et brûlure par l'eau chaude s'écoulant dans la canalisation de circulation et à cet effet des premiers moyens de commande 32 sont prévus pour déclencher l'activation des électrovannes 24 et 25 et l'évacuation de l'eau chaude contenue dans la canalisation de circulation 8 et dans la canalisation aval 6 vers l'égout, préalablement à l'ouverture du robinet de distribution 7. L'appareil comporte également des deuxièmes moyens de commande aptes à interdire l'ouverture du robinet de distribution 7 lorsque l'eau contenue dans la canalisation aval 6 à la sortie de l'échangeur 2 est à une température supérieure à une valeur donnée.

La stérilisation du robinet 7 peut s'effectuer au moyen d'un envoi d'eau chaude stérile à 85°C environ à travers celui-ci, cet envoi pouvant être déclenché préalablement à l'évacuation de l'eau par les premiers moyens de commande, au moyen d'une temporisation ou d'une minuterie.

L'appareil est normalement complété, en tant que besoin, par des dispositifs usuels non représentés comme : vanne de vidange, vanne d'arrêt général, clapet de non-retour, appareil de sécurité, de commande et de mesure contrôlant le manque d'eau, la pression dans le réservoir, les températures, les débits dans les circuits, etc... .

L'appareil s'utilise de préférence de la façon suivante : le réservoir 3 est tout d'abord rempli d'eau de ville, puis cette eau est portée à 90°C par la résistance électrique 5.

Pour stériliser la canalisation aval 6, à l'exception du robinet de distribution 7, on fait circuler, au moyen de la pompe 10, de l'eau chaude à la température d'environ 85°C à 90°C pendant 20 mn environ, dans la canalisation aval 6 et la canalisation de circulation 8. L'eau qui est renvoyée dans le réservoir par l'entrée basse 22 maintient en pression ce dernier.

Après stérilisation de la canalisation aval, l'appareil est apte à distribuer de l'eau stérile et restera constamment, grâce au circuit de circulation, dans des conditions stériles lorsqu'il n'y a pas de demande d'eau stérile et que le robinet de distribution 7 reste fermé.

Pour obtenir de l'eau stérile, l'utilisateur actionne un organe de commande approprié (électrique, manuel, optique, etc...) qui agit sur les premiers et seconds moyens de commande précités, et qui déclenche successivement l'ouverture pendant quelques instants du robinet de distribution 7 pour le stériliser puis l'ouverture des électrovannes 24 et 25 de purge, ce qui permet d'éliminer l'eau chaude stérile de la canalisation 8 du circuit de circulation et de la canalisation aval 6. Ensuite, environ une minute après cette action sur l'organe de commande, les seconds moyens de commande autorisent l'ouverture durable du robinet 7 qui peut ainsi distribuer de l'eau stérile à une température qui ne présente plus de risque de brûlure.

A la fin de la distribution d'eau stérile, le circuit de circulation continue à établir dans la canalisation aval 6 des conditions stériles qui évitent toute contamination microbienne.

Le mitigeur 11 permet de réguler la température d'entrée d'eau de ville dans l'appareil, donc l'échange de chaleur entre les canalisations aval 6 et amont 1 dans l'échangeur 2 et la température finale de distribution d'eau stérile par le robinet 7.

La vanne de réglage de débit 14 permet de limiter le débit maximal d'eau stérile délivré par l'appareil.

L'échangeur 2 est choisi parmi les échangeurs connus (serpentins, serpentin à ailettes, tubes droits, tubes droits à ailettes, échangeurs à plaques, etc...) en fonction des conditions de service (perte de charge, risque d'encrassement, risque de corrosion, nature des matériaux, etc...).

## Revendications

**1/** Appareil pour la production d'eau stérile (ES) à partir d'eau de ville (EV), du type comprenant un circuit d'eau comportant une canalisation amont (1) alimentée en eau de ville, traversant un échangeur de chaleur (2) et débouchant dans un réservoir (3) muni d'une résistance électrique (5) apte à porter l'eau du réservoir à une température de stérilisation et une canalisation aval (6) partant du réservoir, traversant l'échangeur (2) et aboutissant à une sortie d'eau stérile (ES) munie d'un robinet de distribution (7), ledit échangeur de chaleur (2) étant apte à réaliser un échange de chaleur entre ladite canalisation aval (6) et ladite canalisation amont (1), caractérisé en ce que l'appareil comporte un circuit de circulation d'eau chaude stérile comportant une canalisation de circulation (8) partant de ladite canalisation aval à un point de prélèvement (9) situé entre l'échangeur de chaleur (2) et ledit robinet de distribution (7) et débouchant dans le réservoir (3) et une pompe (10) montée dans cette canalisation pour y faire circuler l'eau chaude stérile prélevée dans la canalisation aval (6) lorsque le robinet de distribution (7) est fermé.

**2/** Appareil selon la revendication 1, caractérisé en ce que la canalisation amont (1) est alimentée en eau de ville au moyen d'un mitigeur (11) comportant une entrée (12) d'eau chaude sanitaire (EC) et une entrée (13) d'eau de ville (EV), de façon à permettre un réglage de la température de l'eau de ville alimentant la canalisation amont et la température de sortie de l'eau stérile (ES).

**3/** Appareil selon la revendication 1 ou 2, caractérisé en ce qu'il comporte, partant de la canalisation de circulation en aval de la pompe et en amont d'un clapet anti-retour (21) monté dans la canalisation de circulation, un circuit de purge muni d'une électrovanne (24) débouchant sur une canalisation d'évacuation (30) vers un égout, ainsi qu'une alimentation en eau froide pour refroidir l'eau délivrée par cette électrovanne lors de son rejet dans ladite canalisation d'évacuation.

**4/** Appareil selon l'une des revendications 1 à 3, caractérisé en ce qu'il comporte dans la canalisation amont (1) une vanne (14) de réglage du débit d'alimentation en eau de ville de cette canalisation (1).

**5/** Appareil selon l'une des revendications 1 à 4, caractérisé en ce qu'il comporte des premiers moyens de commande (31) pour déclencher l'évacuation de l'eau chaude stérile contenue dans la canalisation de circulation et dans la canalisation aval vers l'égout, préalablement à l'ouverture du robinet de distribution.

**6/** Appareil selon l'une des revendications 1 à 5, caractérisé en ce qu'il comporte des seconds moyens de commande (32) aptes à interdire l'ouverture du robinet de distribution lorsque l'eau contenue dans la canalisation aval (6) à la sortie de l'échangeur (2) est à une température supérieure à une valeur donnée.
